# EUROPEAN PATENT SPECIFICATION

(11) **EP 3 976 164 B1**
(45) Date of publication and mention of the grant of the patent: **30.04.2025**
(21) Application number: 20727655.1
(22) Date of filing: 27.05.2020
(51) Int. Cl.: A61M 39/22

(54) **HOLDING DEVICE AND APPARATUS FOR AUTOMATED STOPCOCK ACTUATION**
HALTEVORRICHTUNG UND VORRICHTUNG ZUR AUTOMATISCHEN ABSPERRHAHNBETÄTIGUNG
DISPOSITIF DE MAINTIEN ET APPAREIL D'ACTIONNEMENT DE ROBINET D'ARRÊT AUTOMATISÉ

(30) Priority: 29.05.2019 EP 19305683
(43) Date of publication of application: 06.04.2022
(73) Proprietor: Merck Patent GmbH, 64293 Darmstadt (DE)
(72) Inventor: OLIVIER, Stéphane, 67560 Rosheim (FR); SCHMITT, Gaelle, 67710 Wangenbourg-Engenthal (FR); BOUR, Gaetan, 67540 Ostwald (FR)
(74) Representative: Merck Patent Association
(86) International application number: PCT/EP2020/064624
(87) International publication number: WO 2020/239794

(56) References cited:
- EP-A1- 2 221 084
- WO-A1-2017/178156
- WO-A1-2019/094890
- US-A1- 2014 196 792

## Description

The present invention concerns a holding device and an apparatus for automated stopcock actuation, in particular for use in conjunction with disposable multiway stopcocks.

The invention generally relates to the fields of medical care, food analysis or testing but can also find applications in connection with hydroponic plant growth and other applications where disposable stopcocks for setting up flow arrangements for fluids are used.

Disposable stopcocks have been designed to be normally actuated by hand as is the case for most user applications in the medical or any of the other above-mentioned fields. Stopcocks, to which the present invention pertains, are functional components made from plastic material including a valve casing provided with plural external ports, which can be in return formed as male or female luer connectors, sleeves, enteral connectors, barb connectors etc. to be connected to suitable tubing to set up the desired flow arrangement.

The valve casing rotatably receives a valve body formed to selectively establish or block the flow connections between the various ports of the valve casing. The valve body is connected with an external handle at which the user can grip and actuate, i.e. rotate the valve body inside the valve casing between selected positions. Examples of stopcocks of this type can be found in the product portfolio of several suppliers such as Qosina, Nordson Medical, Elcam, Mednet, Smiths Medical.

In some applications it is known to actuate disposable stopcocks by an automated rotary actuator, for example in radiosynthesis preparations, which allows a number of stopcocks in a flow arrangement to be automatically operated according to a predefined schedule without manual interaction. Such an actuator includes a stepper motor, a stopcock-backplate and a coupler connecting the drive shaft of the stepper motor to the handle of the stopcock. It may also include a home-position sensor for detecting a specific position of the handle.

In order to prevent any risks of contamination (chemical, microbial, pathogen etc.) there is a need for safe and low-cost disposable solutions in the above-mentioned fields, promoting the use of disposable tubing sets to set up a flow arrangement for processing fluids and to dispense, mix, aliquot, transform or analyse fluids from different containers, equipment or living organisms (human, plant, cell culture etc.).

Further, multiway-stopcocks have been developed as a low-cost disposable solution for many applications including medical, to select or isolate different flow paths. The design has been optimized for manual actuation and is not truly compatible with automated actuation solutions. In particular, current low-cost disposable stopcocks on the market have not been designed for automation and are not easy to use in conjunction with automated equipment.

WO 2017/178156 A1 stopcock manifold system comprising a manifold (7) having: one or more stop-cocks (2) each comprising at least one finger or handle ( 4); for every stopcock (2), a connection element (5, 10) for connecting a mechanical actuator; and a retaining system designed to ensure that every connection element (5, IO) individually keeps the stopcocks (2) in place.

EP 2 221 084 A1 discloses a manual valve actuator comprises a holder (50) adapted to removably receive a valve mechanism (24) of the medical fluid delivery set, a valve driver (52), and a handle (54) for rotating the valve driver and thereby the valve rotor (34) when the valve mechanism is received in the holder.

WO 2019/094890 A1 discloses a housing assembly comprising: a first housing body; a second housing body pivotally coupled to the first housing body, first and second housing body being moveable relative to each between an open and closed position, wherein the housing assembly forms an interior chamber with one or more openings when in the closed position; a cap configured to be disposed within the interior chamber and configured to receive a complementary connection.

US 2014/196792 A1 discloses a three-way valve case that has a top member, bottom member, closure member, three openings, and a hinge allowing the case to be opened and closed. The case may be adapted to receive a three-way valve. Apart from a case adapted to receive a three-way valve, a three-way valve case apparatus is also disclosed. The apparatus includes a three-way valve having three ports, a base, and an operator; and a case having a top member, bottom member, hinge, openings to receive the ports of the valve, and a closure member to support the closed state of the case.

It is the object of the present invention to provide a holding device and an apparatus for automated stopcock actuation which can solve one or more of the following existing problems:
provide easy accessibility for placing the stopcocks in the holding device, preferably by a single step manual loading and unloading of the stopcock;
provide accurate positioning of the stopcock in the holding device, in particular with respect to an accurate centered and angular position of the stopcock body when loaded;
provide improved security preventing in particular an inadvertent removal of the stopcock except for specific loading/unloading position; and
provide a possibility of detecting distinct states of the holding device, i.e. with the existence of the stopcock in the holding device and/or a specific rotation position.

To solve at least some of the above problems, the present invention provides a holding device for automated stopcock actuation with the features of claim 1. Preferred embodiments are defined in the dependent claims.

Further, the invention also provides an apparatus for automated stopcock actuation as defined in claim 14 and including the holding device of the present invention.

A holding device for automated stopcock actuation in particular comprises a base body; characterized in that the holding device further comprises a driver rotatably received at the base body, wherein the holding device has a receptacle for removably receiving at least a part of a stopcock and configured such that the driver can engage with and actuate a handle of the stopcock received in the receptacle upon rotation of the driver, and a first fixation member that is configured to allow selective fixation of the stopcock in the receptacle, wherein the first fixation member is configured to allow placement of the stopcock into the receptacle in a defined rotation position of the driver and prevent removal of the stopcock from the receptacle upon a relative rotation, between the driver and the base body, and wherein the first fixation member comprises a lip, preferably on the base body, so as to partially overlap the receptacle, preferably a part where the handle of the stopcock is placed, and a notch in the lip arranged so as to prevent removal of the stopcock placed in the receptacle over a defined rotation range of the driver and allow removal/placement of the stopcock at a defined rotation position where the notch is aligned with the receptacle.

Preferably, the receptacle has a protrusion configured to engage with a part of the stopcock, preferably a pin configured to be inserted into a bore concentric with a rotation axis of the handle of the stopcock.

Preferably, the holding device has one or more second fixation members respectively configured to releasably hold a tubing section leading to a port of the stopcock.

Preferably, plural second fixation members are distributed about a circumference of the holding device.

Preferably, at least one of the second fixation member/members is configured to allow a releasable form-locking engagement, preferably a snap-fit engagement with the tubing section.

Preferably, the at least one of the second fixation member/members is respectively formed by a slot formed in the base part and configured to hold the tubing section leading to the port of the stopcock.

Preferably, the holding device further comprises a biasing element configured to bias the stopcock placed in the receptacle into engagement with the first fixation member.

Preferably, the driver is configured to be coupled to an external rotary actuator.

Preferably, the holding device further comprises a sensor device for detecting a rotation position and/or presence of the stopcock in the receptacle of the holding device.

Preferably, the base body is configured to be removably attached to an adjacent base body of another holding device to form an array.

Preferably, the base body comprises plural receptacles and associated independent drivers arranged in an array.

An apparatus for automated stopcock actuation according to the invention comprises one or more holding device/devices according to the invention, and one or more rotary actuator/actuators for engaging with the driver/drivers of the holding device/devices and configured to drive the driver/drivers for rotation.

Preferably, the apparatus is configured to allow releasable attachment of the holding device/devices.

The following is the description of preferred embodiments of the holding device of the present invention in conjunction with the attached drawing in which:
Figure 1 is a perspective external view of a holding device according to an embodiment and a stopcock arrangement before the latter is mounted in the former;
Figure 2A is a perspective view of the holding device of an embodiment in the state where the stopcock is mounted and Figure 2B is a cross-sectional view of the rotation axis of the stopcock shown in Figure 2A;
Figure 3A is a front view of the embodiment of the holding device of Figure 2A indicating a rotation position creating a selective fixation of the stopcock in the holding device and Figure 3B is a cross-sectional perspective view along a bent line in Figure 3A;
Figure 4 is a side view of a magnified detail of the embodiment of Figure 1 to explain an example of the second fixation device for tubing of the stopcock;
Figures 5A and 5B show two examples of arrangements of holding devices for plural stopcocks; and
Figures 6A and 6B are examples of sensor arrangements in the holding device of the present invention.

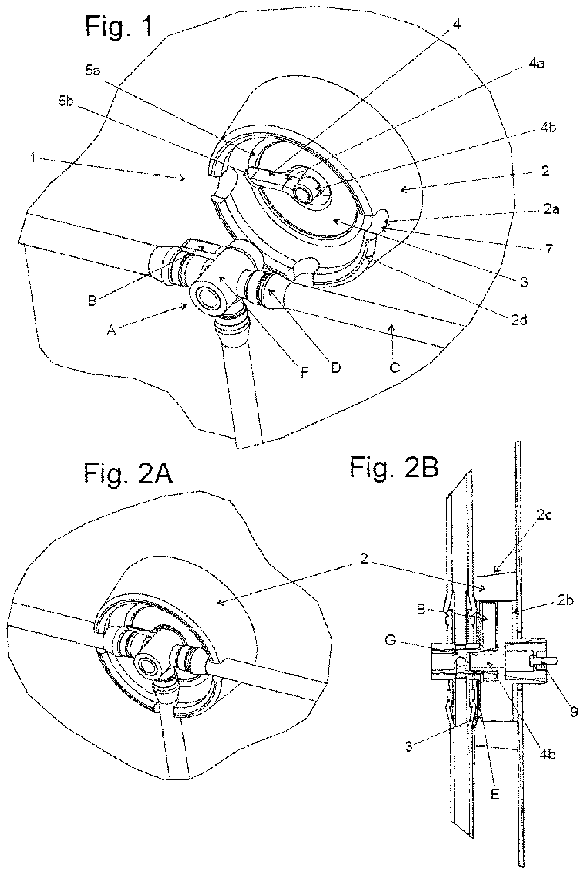

The holding device 1 for automated stopcock actuation of the embodiment shown in the figures 1 to 4 comprises a base body 2 and a driver 3 rotatably mounted to the base body 2. The holding device 1 has a receptacle 4 for removably receiving at least a part of a disposable stopcock A and configured such that the driver 3 can engage with and actuate a handle B of the valve body G of the valve casing F of the stopcock A once received in the receptacle 4, preferably by rotation, of the driver 3.

The receptacle 4 in the driver 3 can be more or less conformed to the outer shape of the handle B and can accordingly include an accommodation space for a single handle lever or an accommodation space for plural handle levers, for example in the form of a cross- or Y-arrangement. In order to adapt the holding device 1 to different stopcock models the driver can be provided with exchangeable elements having different receptacles (not shown) in order to be able to configure the holding device to operate with different types of stopcocks. The receptacle does not necessarily have to correspond to the entire outer shape or footprint of the stopcock or its handle and it is sufficient to receive only a part of the stopcock and its handle so as to engage therewith and transmit the rotation force of the driver to the handle. In other words, the receptacle does not have to have a fully complementary cavity that corresponds to the complete outer shape or contour of the handle B.

The base body 2 is a fixed element that can have various shapes or configurations, some of which will be described further below.

In the embodiment shown in Figures 1 to 3 the base body 2 is a round cylinder with inclined outer sidewalls 2c and an internal opening 2b configured to receive the driver 3 so as to allow rotation of the driver 3 relative to the base body 2. The receptacle 4 is formed in the driver 3 so as to allow insertion of the stopcock A "upside down" i.e. with the handle B first as shown in Figure 1.

The base body 2 has, at the periphery surrounding the opening 2b receiving the driver 3, a lip 5a extending about the circumference of the driver 3 and preventing a removal of the driver from the internal opening 2b to the front side of the base body. The circumferential lip 5a is furthermore formed so as to also overlap a part of the receptacle 4 where the handle B of the stopcock A is placed. At a distinct angular position (9 o'clock in the representation of Figure 1) the lip 5a is provided with a notch 5b so that, when the receptacle 4 of the driver is aligned with the position of the notch 5b, the stopcock A or at least a part of the handle B can be placed in the receptacle (see Figures 1 and 2A and 2B)

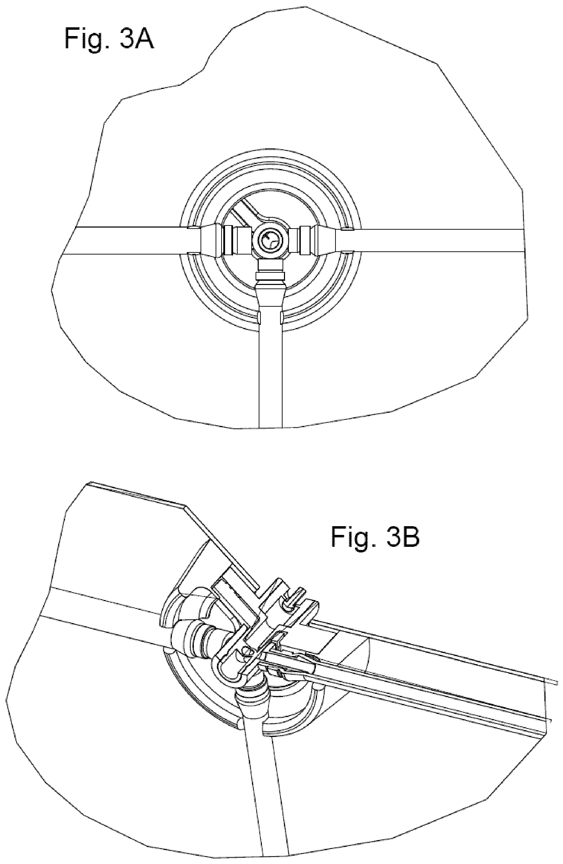

Once the stopcock A (or handle B) is arranged in the receptacle 4 to an insertion position or depth so that the handle B is located, in the insertion direction, below the protruding circumferential lip 5a, rotation of the driver 3 relative to the base body 2 brings the radially outer part of the lever B, where the circumferential lip 5a overlaps the receptacle 4, below the inside circumferential axial surface of the lip facing the opening 2b in the base body 2 (see Figures 3A and B), thereby preventing removal of the stopcock from the receptacle 4 to the front side of the holding device by a form-locking engagement at the outer end portion of the handle B with the circumferential lip 5a.

Accordingly, the interaction between the lip 5a, the notch 5b in the lip 5a and the receptacle 4 of the driver 3 may represent a first fixation member 5 that allows selective fixation of the stopcock in the receptacle and that allows placement of the stopcock into the receptacle in a defined rotation position of the driver and prevents removal of the stopcock from the receptacle after and over a certain relative movement, i.e. rotation, between the driver and the base body. The initial rotation of the driver over a certain angular range bringing the lever under the lip 5a for fixing the stopcock in the holding device can be manually made or can be a locking function of an apparatus operating the driver for the necessary rotation range.

In a modification that is not shown in the drawing the lip 5a, and optionally the notch 5b, may - instead of being formed in, fixed to or integrated with the base body 2 - be part of a separate ring element that is rotatably attached to the base body. In this case the ring element alone can be rotated relative to the driver and the base body to "lock" the lever of the stopcock in the receptacle.

As shown in Figures 2B and 3B the receptacle 4 has preferably a protrusion 4b configured to engage with a part of the stopcock, preferably in the form of a pin configured to be inserted and tightly fitted into a bore E concentric with a rotation axis of the valve body G of the stopcock A. This protrusion has a centering function and also preferably prevents, due to its protruding height, a tilting movement of the stopcock in the receptacle so that, in conjunction with the form-locking engagement of the outer end of the handle with the circumferential lip 5a, a removal of the stopcock from the receptacle is prevented in essentially all rotation positions other than the position where the receptacle is aligned with the notch 5b in the lip 5a. The central protrusion 4b - if present - also guides and centers the stopcock during insertion and/or fully immobilizes the stopcock handle when in the receptacle in conjunction with the overlapping lip.

The central protrusion 4b can be omitted if the valve body G of the stopcock used is not provided with a corresponding bore E. It can also be replaced by a protrusion engaging on the outside of the valve body G.

It goes without saying that in a case, where the handle of a specific stopcock has two or more protrusions or lugs in a X(cross)- or Y-arrangement, the lip 5a would be provided with a corresponding number and arrangement of notches 5b to allow the insertion of the handle into the receptacle 4 at a specific loading/unloading position and to prevent, upon relative rotation, the removal as described above.

The holding device 1 preferably has, as shown in the Figures, one or more second fixation members 7 which are respectively configured to releasably hold a tubing section C leading to a port D of the stopcock A. These second fixation members 7 are distributed about the circumference of the holding device 1, in particular of the base body 2, and they are aligned with the direction of the ports D of the stopcock A used. The holding device 1 may contain several such second fixation members 7 as needed for a particular stopcock in order to accommodate different multiway stopcock concepts.

One or more or preferably all of these second fixation members 7 is/are configured to allow a releasable form-locking engagement with the tubing C, preferably a snap-fit engagement. This can be realized in multiple ways.

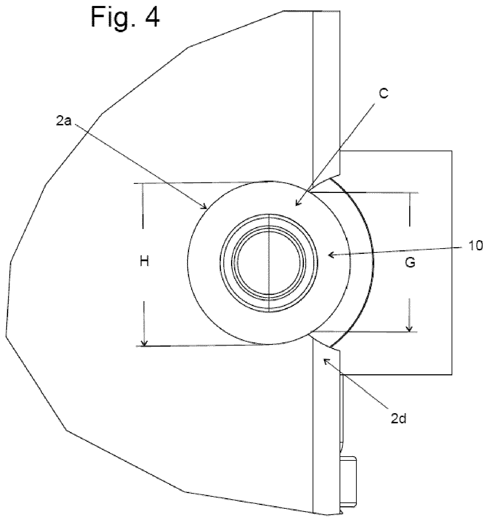

A particularly simple structure of the second fixation member 7 shown in the Figures comprises a slot 2a formed in an outer, axially protruding peripheral rim 2d of the base body 2 and formed so as to receive and hold the tubing section C leading to one of the ports D of the stopcock A. As shown in Figure 4 a simple arrangement can be realized, in case the tubing C is made from an elastomer or a deformable plastic, in that the slot 2a is formed with an inner diameter H corresponding essentially to the outer diameter of the tube C and extending over an arc section that is greater than 180°, leaving an aperture 10 facing towards the axial front side of the device and having a width G that is smaller than the tube diameter H. This enables an easy insertion of the flexible tubing being temporarily deformed when passing the aperture 10 and recovering its full diameter in the final position inside the slot 2a.

Other arrangements of the second fixation member 7 are possible. One particularly preferred variant that is not shown in the drawing comprises a mechanical concept that is similar to the releasable holding of the lever in the receptacle by means of the interaction with the lip 5a of the base body in that a rotatable further ring element is provided on the front side of the base body, for example on a radially inner or outer side of the peripheral rim 2d, and provided with notches aligned with the slots 2a and dimensioned so that they allow, in an insertion position, insertion of the tubing sections into the slots. Relative rotation of such ring element to the base body could then selectively close the axial insertion-side of the slots and prevent removal of the tubing from the slots. The rotation of such ring element relative to the base body can be independent or combined with the rotation of the driver or the other ring element of the first fixation member (not shown).

The fixation of the tubings C in the second fixation members 7 may be sufficient, in the broadest concept, to hold the stopcock A in the receptacle 4 of the holding device 1 when the fluid system is set up even if the driver 3 is not yet rotated to bring the portion of the handle B below the lip 5a. The rotation of the driver 3 is in this case not necessary to hold the stopcock A but provides additional and "unremovable" fixation of the stopcock in operation.

The holding device may furthermore include a biasing element (not shown), for example in the form of a spring or elastic member, configured to bias the stopcock A placed in the receptacle 4 into engagement with the first fixation member 5, i.e. the circumferential lip 5a in the embodiment, in that such biasing element is arranged rearward of the driver in the opening of the base body and pushing the driver against the insertion direction of the stopcock.

The driver 3 may be configured to be coupled to an external rotary actuator 9 at the opposite side from the cavity 4 (see Figures 2B, 3B).

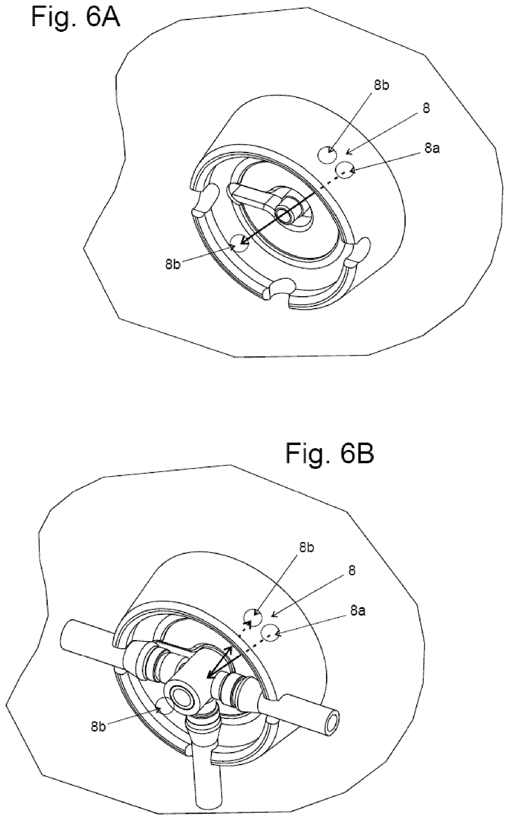

As shown in Figures 6A and 6B the holding device 1 may furthermore include a sensor device 8 for detecting a rotation position and/or presence of the stopcock A in the receptacle 4 of the holding device 1. Such sensor device 8 may be an optical sensor working either by transmission or reflection and may include an emission part 8a and one or more detection part/parts 8b arranged relative to the emission part 8a so as to be able to receive a transmitted or reflected portion of the light emitted from the emission part (Figure 6A showing an example of a presence detection relying on transmission or non-transmission and Figure 6B showing an example relying on the reflection). Depending on the arrangement of the elements of the sensor device 8 distinct rotation positions can be detected as well.

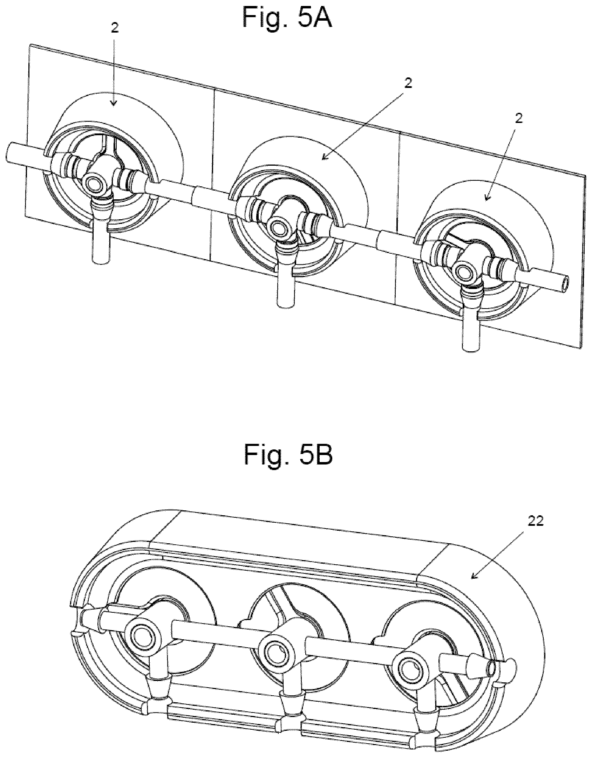

The Figures 5A and 5B show two examples of creating an array of receptacles for receiving multiple stopcocks in an array composing a manifold. The solution of Figure 5A includes plural independent base bodies 2 with their corresponding drivers in an arrangement next to each other or configured to be removably attached to each other at the base bodies to form an integral unit. In the variant of Figure 5B the base body 22 is enlarged and receives a plurality of independent drivers and receptacles arranged in a predetermined configuration.

The invention also pertains to an apparatus for automated stopcock actuation using one or more of the holding devices 1 described in this application and including one or more rotary actuators for engaging with the drivers 3 of the holding devices and configured to drive the drivers for rotation, normally in conjunction with an internal or external controller. The actuators can be step-motors, servomotors, magnetic actuators or other rotating transmission systems.

To increase the flexibility to accommodate different flow arrangements, the apparatus may be configured to allow releasable attachment of the holding devices to configure the apparatus to different arrangements.

## Claims

1. A holding device (1) for automated stopcock actuation, comprising:
a base body (2);
**characterized in that** the holding device (1) further comprises a driver (3) rotatably received at the base body (2),
wherein the holding device (1) has a receptacle (4) for removably receiving at least a part of a stopcock (A) and configured such that the driver (3) can engage with and actuate a handle (B) of the stopcock (A) received in the receptacle (4) upon rotation of the driver (3), and
a first fixation member (5) that is configured to allow selective fixation of the stopcock (A) in the receptacle (4),
wherein the first fixation member (5) is configured to allow placement of the stopcock (A) into the receptacle (4) in a defined rotation position of the driver (3) and prevent removal of the stopcock (A) from the receptacle (4) upon a relative rotation, between the driver (3) and the base body (2), and
wherein the first fixation member (5) comprises a lip (5a), so as to partially overlap the receptacle (4), and a notch (5b) in the lip (5a) arranged so as to prevent removal of the stopcock (A) placed in the receptacle (4) over a defined rotation range of the driver (3) and allow removal/placement of the stopcock (A) at a defined rotation position where the notch (5b) is aligned with the receptacle (4).

2. The holding device according to claim 1, wherein the first fixation member (5) comprises a lip (5a) on the base body.

3. The holding device according to claim 1 or claim 2, wherein the first fixation member (5) comprises a lip (5a) so as to partially overlap **a part of** the receptacle where the handle (B) of the stopcock (A) is placed.

4. The holding device (1) according to any one of claims 1 to 3, wherein the receptacle (4) has a protrusion (4b) configured to engage with a part of the stopcock (A), preferably a pin configured to be inserted into a bore (E) concentric with a rotation axis of the handle (B) of the stopcock (A).

5. The holding device (1) according to any one of claims 1 to 4, wherein the holding device (1) has one or more second fixation members (7) respectively configured to releasably hold a tubing section (C) leading to a port (D) of the stopcock (A).

6. The holding device (1) according to claim 5, wherein plural second fixation members (7) are distributed about a circumference of the holding device (1).

7. The holding device (1) according to claim 5 or 6, wherein at least one of the second fixation member/members (7) is configured to allow a releasable form-locking engagement, preferably a snap-fit engagement with the tubing section (C).

8. The holding device (1) according to claim 7, wherein the at least one of the second fixation member/members (7) is respectively formed by a slot (2a) formed in the base part (2) and configured to hold the tubing section (C) leading to the port (D) of the stopcock (A).

9. The holding device (1) according to any one of claims 1 to 8, further comprising a biasing element configured to bias the stopcock (A) placed in the receptacle (4) into engagement with the first fixation member (5).

10. The holding device (1) according to any one of claims 1 to 9, wherein the driver (3) is configured to be coupled to an external rotary actuator (9).

11. The holding device (1) according to any one of claims 1 to 10, comprising a sensor device (8) for detecting a rotation position and/or presence of the stopcock (A) in the receptacle (4) of the holding device (1).

12. The holding device according to any one of claims 1 to 11, wherein the base body (2) is configured to be removably attached to an adjacent base body (2) of another holding device (1) to form an array.

13. The holding device (1) according to any one of claims 1 to 11, wherein the base body (2) comprises plural receptacles (4) and associated independent drivers (3) arranged in an array.

14. An apparatus for automated stopcock actuation, comprising:
one or more holding device/devices (1) as defined in any one of claims 1 to 13, and
one or more rotary actuator/actuators for engaging with the driver/drivers (3) of the holding device/devices (1) and configured to drive the driver/drivers (3) for rotation.

15. The apparatus for automated stopcock actuation according to claim 14, wherein the apparatus is configured to allow releasable attachment of the holding device/devices (1).

## Patentansprüche

1. Haltevorrichtung (1) zur automatischen Absperrhahnbetätigung, umfassend:
einen Grundkörper (2);
**dadurch gekennzeichnet, dass** die Haltevorrichtung (1) ferner einen Mitnehmer (3) umfasst, der drehbar am Grundkörper (2) aufgenommen ist,
wobei die Haltevorrichtung (1) eine Aufnahme (4) zum entnehmbaren Aufnehmen mindestens eines Teils eines Absperrhahns (A) aufweist und so konfiguriert ist, dass der Mitnehmer (3) einen Griff (B) des in der Aufnahme (4) aufgenommenen Absperrhahns (A) in Eingriff nehmen und diesen betätigen kann, wenn der Mitnehmer (3) gedreht wird, und
ein erstes Fixierelement (5), das dafür konfiguriert ist, eine selektive Fixierung des Absperrhahns (A) in der Aufnahme (4) zu ermöglichen,
wobei das erste Fixierelement (5) dafür konfiguriert ist, das Platzieren des Absperrhahns (A) in der Aufnahme (4) in einer definierten Drehposition des Mitnehmers (3) zu ermöglichen und die Entnahme des Absperrhahns (A) aus der Aufnahme (4) bei einer relativen Drehung zwischen dem Mitnehmer (3) und dem Grundkörper (2) zu verhindern, und
wobei das erste Fixierelement (5) eine Lippe (5a), um die Aufnahme (4) teilweise zu überlappen, und eine Kerbe (5b) in der Lippe (5a), die angeordnet ist, um die Entnahme des in der Aufnahme (4) platzierten Absperrhahns (A) über einen definierten Drehbereich des Mitnehmers (3) zu verhindern und die Entnahme/Platzierung des Absperrhahns (A) in einer definierten Drehposition, in der die Kerbe (5b) mit der Aufnahme (4) ausgerichtet ist, zu ermöglichen, umfasst.

2. Haltevorrichtung nach Anspruch 1, wobei das erste Fixierelement (5) eine Lippe (5a) am Grundkörper umfasst.

3. Haltevorrichtung nach Anspruch 1 oder Anspruch 2, wobei das erste Fixierelement (5) eine Lippe (5a) umfasst, um **einen Teil der** Aufnahme, wo der Griff (B) des Absperrhahns (A) platziert ist, teilweise zu überlappen.

4. Haltevorrichtung (1) nach irgendeinem der Ansprüche 1 bis 3, wobei die Aufnahme (4) einen Vorsprung (4b) aufweist, der dafür konfiguriert ist, einen Teil des Absperrhahns (A) in Eingriff zu nehmen, vorzugsweise einen Stift, der dafür konfiguriert ist, in eine Bohrung (E) eingesetzt zu werden, die konzentrisch zu einer Drehachse des Griffs (B) des Absperrhahns (A) ist.

5. Haltevorrichtung (1) nach irgendeinem der Ansprüche 1 bis 4, wobei die Haltevorrichtung (1) ein oder mehrere zweite Fixierelemente (7) aufweist, die jeweils dafür konfiguriert sind, einen zu einem Anschluss (D) des Absperrhahns (A) führenden Schlauchabschnitt (C) lösbar festzuhalten.

6. Haltevorrichtung (1) nach Anspruch 5, wobei mehrere zweite Fixierelemente (7) über einen Umfang der Haltevorrichtung (1) verteilt sind.

7. Haltevorrichtung (1) nach Anspruch 5 oder 6, wobei mindestens eines des/der zweiten Fixierelementes/Fixierelemente (7) dafür konfiguriert ist, einen lösbaren formschlüssigen Eingriff, vorzugsweise eine Schnappverbindung, mit dem Schlauchabschnitt (C) zu ermöglichen.

8. Haltevorrichtung (1) nach Anspruch 7, wobei das mindestens eine des/ der zweiten Fixierelementes/Fixierelemente (7) jeweils durch einen im Grundteil (2) ausgebildeten Schlitz (2a) gebildet wird und dafür konfiguriert ist, den zum Anschluss (D) des Absperrhahns (A) führenden Schlauchabschnitt (C) festzuhalten.

9. Haltevorrichtung (1) nach irgendeinem der Ansprüche 1 bis 8, ferner umfassend ein Vorspannelement, das dafür konfiguriert ist, den in der Aufnahme (4) platzierten Absperrhahn (A) in Eingriff mit dem ersten Fixierelement (5) vorzuspannen.

10. Haltevorrichtung (1) nach irgendeinem der Ansprüche 1 bis 9, wobei der Mitnehmer (3) dafür konfiguriert ist, an einen externen Drehantrieb (9) gekoppelt zu werden.

11. Haltevorrichtung (1) nach irgendeinem der Ansprüche 1 bis 10, umfassend eine Sensorvorrichtung (8) zum Erfassen einer Drehposition und/oder eines Vorhandenseins des Absperrhahns (A) in der Aufnahme (4) der Haltevorrichtung (1).

12. Haltevorrichtung nach irgendeinem der Ansprüche 1 bis 11, wobei der Grundkörper (2) dafür konfiguriert ist, abnehmbar an einem benachbarten Grundkörper (2) einer anderen Haltevorrichtung (1) befestigt zu werden, um eine Anordnung zu bilden.

13. Haltevorrichtung (1) nach irgendeinem der Ansprüche 1 bis 11, wobei der Grundkörper (2) mehrere Aufnahmen (4) und zugehörige unabhängige Mitnehmer (3) umfasst, die in einer Anordnung angeordnet sind.

14. Apparatur zur automatischen Absperrhahnbetätigung, umfassend:
eine oder mehrere Haltevorrichtung/Haltevorrichtungen (1) wie in irgendeinem der Ansprüche 1 bis 13 definiert, und
einen oder mehrere Drehantrieb/Drehantriebe, um den/die Mitnehmer (3) der Haltevorrichtung/Haltevorrichtungen (1) in Eingriff zu nehmen, und dafür konfiguriert, den/die Mitnehmer (3) zur Drehung anzutreiben.

15. Apparatur zur automatischen Absperrhahnbetätigung nach Anspruch 14, wobei die Apparatur dafür konfiguriert ist, eine lösbare Befestigung der Haltevorrichtung/Haltevorrichtungen (1) zu ermöglichen.

## Revendications

1. Dispositif de maintien (1) pour l'actionnement automatisé d'un robinet d'arrêt, comprenant :
un corps de base (2) ;
**caractérisé en ce que** le dispositif de maintien (1) comprend en outre un dispositif d'entraînement (3) reçu à rotation au niveau du corps de base (2),
dans lequel le dispositif de maintien (1) comporte un réceptacle (4) pour recevoir de façon mobile et amovible au moins une partie d'un robinet d'arrêt (A) et configuré de telle sorte que le dispositif d'entraînement (3) puisse être engagé avec une poignée (B) du robinet d'arrêt (A) reçu dans le réceptacle (4) et puisse actionner celle-ci suite à la rotation du dispositif d'entraînement (3), et
un premier élément de fixation (5) qui est configuré pour permettre la fixation sélective du robinet d'arrêt (A) dans le réceptacle (4),
dans lequel le premier élément de fixation (5) est configuré pour permettre le placement du robinet d'arrêt (A) dans le réceptacle (4) dans une position de rotation définie du dispositif d'entraînement (3) et pour empêcher l'enlèvement du robinet d'arrêt (A) hors du réceptacle (4) suite à une rotation relative entre le dispositif d'entraînement (3) et le corps de base (2), et
dans lequel le premier élément de fixation (5) comprend une lèvre (5a) qui est telle qu'elle chevauche partiellement le réceptacle (4), et une encoche (5b) dans la lèvre (5a) qui est agencée de manière à empêcher l'enlèvement du robinet d'arrêt (A) placé dans le réceptacle (4) sur une plage de rotation définie du dispositif d'entraînement (3) et à permettre l'enlèvement/le placement du robinet d'arrêt (A) en une position de rotation définie dans laquelle l'encoche (5b) est alignée avec le réceptacle (4).

2. Dispositif de maintien selon la revendication 1, dans lequel le premier élément de fixation (5) comprend une lèvre (5a) sur le corps de base.

3. Dispositif de maintien selon la revendication 1 ou la revendication 2, dans lequel le premier élément de fixation (5) comprend une lèvre (5a) qui est telle qu'elle chevauche partiellement **une partie du** réceptacle au niveau de laquelle la poignée (B) du robinet d'arrêt (A) est placée.

4. Dispositif de maintien (1) selon l'une quelconque des revendications 1 à 3, dans lequel le réceptacle (4) comporte une protubérance (4b) configurée pour être engagée avec une partie du robinet d'arrêt (A), de préférence une broche configurée pour être insérée à l'intérieur d'un alésage (E) concentrique par rapport à un axe de rotation de la poignée (B) du robinet d'arrêt (A).

5. Dispositif de maintien (1) selon l'une quelconque des revendications 1 à 4, dans lequel le dispositif de maintien (1) comporte un ou plusieurs seconds éléments de fixation (7) respectivement configurés pour maintenir de façon libérable une section de tubage (C) aboutissant à un orifice (D) du robinet d'arrêt (A).

6. Dispositif de maintien (1) selon la revendication 5, dans lequel plusieurs seconds éléments de fixation (7) sont répartis le long d'une circonférence du dispositif de maintien (1).

7. Dispositif de maintien (1) selon la revendication 5 ou 6, dans lequel au moins l'un du/des second(s) élément(s) de fixation (7) est configuré pour permettre un engagement du type blocage libérable, de préférence un engagement par emboîtement par pression avec la section de tubage (C).

8. Dispositif de maintien (1) selon la revendication 7, dans lequel au moins l'un du/des second(s) élément(s) de fixation (7) est respectivement formé par une fente (2a) formée dans la partie de base (2) et configurée pour maintenir la section de tubage (C) aboutissant à l'orifice (D) du robinet d'arrêt (A).

9. Dispositif de maintien (1) selon l'une quelconque des revendications 1 à 8, comprenant en outre un élément de poussée configuré pour pousser le robinet d'arrêt (A) placé dans le réceptacle (4) en engagement avec le premier élément de fixation (5).

10. Dispositif de maintien (1) selon l'une quelconque des revendications 1 à 9, dans lequel le dispositif d'entraînement (3) est configuré pour être couplé à un actionneur rotatif externe (9).

11. Dispositif de maintien (1) selon l'une quelconque des revendications 1 à 10, comprenant un dispositif de capteur (8) pour détecter une position en rotation et/ou la présence du robinet d'arrêt (A) dans le réceptacle (4) du dispositif de maintien (1).

12. Dispositif de maintien selon l'une quelconque des revendications 1 à 11, dans lequel le corps de base (2) est configuré pour être lié de façon mobile et amovible à un corps de base adjacent (2) d'un autre dispositif de maintien (1) pour former un réseau.

13. Dispositif de maintien (1) selon l'une quelconque des revendications 1 à 11, dans lequel le corps de base (2) comprend plusieurs réceptacles (4) et plusieurs dispositifs d'entraînement indépendants associés (3) agencés selon un réseau.

14. Appareil pour l'actionnement automatisé d'un robinet d'arrêt, comprenant :
un ou plusieurs dispositifs de maintien (1) tels que définis selon l'une quelconque des revendications 1 à 13, et
un ou plusieurs actionneurs rotatifs pour être engagés avec le(s) dispositif(s) d'entraînement (3) du/des dispositif(s) de maintien (1) et configurés pour entraîner le(s) dispositif(s) d'entraînement (3) en rotation.

15. Appareil pour l'actionnement automatisé d'un robinet d'arrêt selon la revendication 14, dans lequel l'appareil est configuré pour permettre la liaison libérable du/des dispositif(s) de maintien (1).
